# EUROPEAN PATENT APPLICATION

(11) **EP 1 219 633 A1**
(43) Date of publication of application: **03.07.2002**
(21) Application number: 00964672.0
(22) Date of filing: 04.10.2000
(51) Int. Cl.: C07K 5/075, C07K 1/113, C07C 47/277, C07C 43/23, C07C 45/41, C07C 45/64, C07C 59/64, C07K 1/14, A23L 1/236

(54) **PROCESS FOR PRODUCING ASPARTAME DERIVATIVE, CRYSTALS THEREOF, NOVEL PRODUCTION INTERMEDIATES THEREFOR AND PROCESS FOR PRODUCING THE INTERMEDIATE**

(30) Priority: 08.10.1999 JP 28820799; 08.10.1999 JP 28820899; 15.10.1999 JP 29440999; 18.11.1999 JP 32809999
(71) Applicant: Ajinomoto Co., Inc., Tokyo 104-8315 (JP)
(72) Inventor: KAWAHARA,Shigeru Amino Scien.Lab.,Ajinomoto Co.Inc, Kanagawa-ken 210-0801 (JP); NAGASHIMA,Kazutaka Amino Scie.Lab.Ajinomoto Co.Inc, Kanagawa-ken 210-0801 (JP); MORI,Kenichi Amino Sci.Lab.Ajinomoto Co.Inc., Kanagawa-ken 210-0801 (JP); TAKEMOTO,Tadashi Amino Sci.Lab.Ajinomoto Co.Inc., Kanagawa-ken 210-0801 (JP); ONO,Eriko Amino Sci.Lab. Ajinomoto Co.Inc., Kanagawa-ken 210-0801 (JP)
(74) Representative: Nicholls, Kathryn Margaret
(86) International application number: JP0006933
(87) International publication number: WO0127142

(57) **Abstract**

3-(3-Hydroxy-4-methoxyphenyl)-3-methylbutylaldehyde is produced by subjecting 3-(3-hydroxy-4-methoxyphenyl)-3-methylbutyric acid to a reaction for converting a carboxyl group into a formyl group, whereby the carboxyl group in the butyric acid derivative is converted to a formyl group. Such 3-(3-hydroxy-4-methoxyphenyl)-3-methylbutyric acid can be obtained by reacting 2-halogenoanisole with 3-methylcrotonic acid to produce 3-(3-halogeno-4-methoxyphenyl)-3-methylbutyric acid, and converting the halogen atom in the thus produced butyric acid derivative into a hydroxyl group.

N-[N-[3-(3-hydroxy-4-methoxyphenyl)-3-methylbutyl]-L-α-aspartyl]-L-phenylalanine 1-methyl ester useful as a sweetener having a high degree of sweetness can be easily and efficiently derived on an industrial scale from the thus obtained aldehyde derivative in the reductive alkylation reaction with aspartame. Therefore, the aldehyde derivative is highly excellent as an intermediate for the production of the sweetener.

## Description

### Technical Field

The present invention relates to a novel process for production of an aspartyl dipeptide ester derivative, such as N-[N-[3-(3-hydroxy-4-methoxyphenyl)-3-methylbutyl]-L-α-aspartyl]-L-phenylalanine 1-methyl ester important as a sweetener having a high degree of sweetness.

Further, the present invention relates to a process for production of high purity N-[N-[3-(3-hydroxy-4-methoxyphenyl)-3-methylbutyl]-L- α-aspartyl]-L-phenylalanine 1-methyl ester important as a sweetener having a high degree of sweetness. Specifically, it relates to a process for purification of said compound containing various impurity, to a process for production of said compound with high purity comprising a particularly excellent and conventional method for crystallization, and to the crystal thereof.

In addition, the present invention relates to a process for production of the above N-[N-[3-(3-hydroxy-4-methoxyphenyl)-3-methylbutyl]-L- α-aspartyl]-L-phenylalanine 1-methyl ester, and to a novel aldehyde derivative useful as an intermediate for the production thereof, to a process for production thereof, to an use thereof as the intermediate for the production, and to a novel intermediate for the production thereof.

### Background Art

In recent years, as eating habits have been improved to a high level, fatness caused by excessive sugar intake and diseases accompanied by fatness have been at issue. Accordingly, the development of a low-calory sweetener (sweetening agent) that replaces sugar has been in demand. As a sweetener that has been widely used at present, there is aspartame which is excellent in safety and quality of sweetness, and however, is somewhat problematic in stability.

Under these background, N-[N-[3-(3-hydroxy-4-methoxyphenyl)-3-methylbutyl]-L- α-aspartyl]-L-phenylalanine 1-methyl ester represented by the following has been found as a sweetener which is excellent in stability and moreover is better by far in degree of sweetness, i.e., has the advantage in cost per sweet taste, by a part of the present inventors and the like.

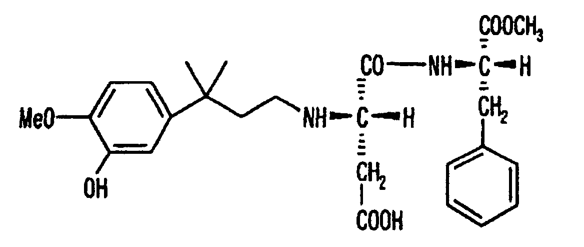

### Problem to be solved by Invention

Because sweeteners are mainly consumed as their objective by a person in the form of foods or pharmaceutical preparations using the same, they should be purified in the process whereby a high purity substance not containing practically any impurity and decomposed material can be obtained. Further, in case of a peptide based sweetener which is easy to be decomposed comparatively, such as the object compound in the present invention, it is desirable that an ultimate form therefor much more excellent in stability is prepared, to prevent from decomposition after shipment of finished product.

In addition, as a process for production of N-[N-[3-(3-hydroxy-4-methoxyphenyl)-3-methylbutyl]-L-α-aspartyl]-L-phenylalanine 1-methyl ester important as a sweetener described above, a process for alkylating reductively β-O-benzyl- α-L-aspartyl-L-phenylalanine methyl ester with 3-(3-benzyloxy-4-methoxyphenyl)-3-methylbutylaldehyde and NaB(OAc)₃H, followed by removing the benzyl group of a protecting group therefrom, and the like were thought up among some ideas of the present inventors. However, 3-(3-benzyloxy-4-methoxyphenyl)-3-methylbutylaldehyde is synthesized through 7 stages of reactions starting from the 3-hydroxy-4-methoxyacetophenone, as shown in the following reaction process 1, and therefore the compound is not an industrially profitable ground substance for reaction.

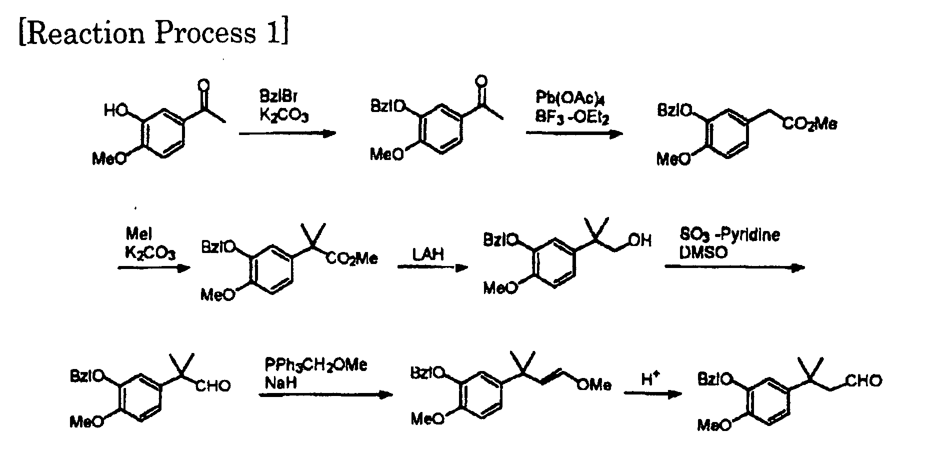

The problem to be solved by the present invention, is to provide a process for producing industrially and efficiently N-[N-[3-(3-hydroxy-4-methoxyphenyl)-3-methylbutyl]-L-α-aspartyl]-L-phenylalanine 1-methyl ester excellent as a sweetener having a high degree of sweetness, and particularly an intermediate for the production therefor or a conventional process for the production thereof. It is also to provide a practical and industrial process for purifying the object compound described above, particularly a process for obtaining the object compound in the crystalline form at a high purity separated from moity impurity therein, and crystals which are in the ultimate form much more excellent in stability as a finished product.

### Disclosure of Invention

The present inventors have earnestly investigated to solve the problem described above, and as a result found that an aspartyl dipeptide ester derivative as the object compound, represented by the following general formula (2) can be easily obtained in the process for alkylating reductively aspartame with an aldehyde represented by the following general formula (1), preferably in the presence of catalyst, more preferably for reacting the above in the presence of hydrogen.

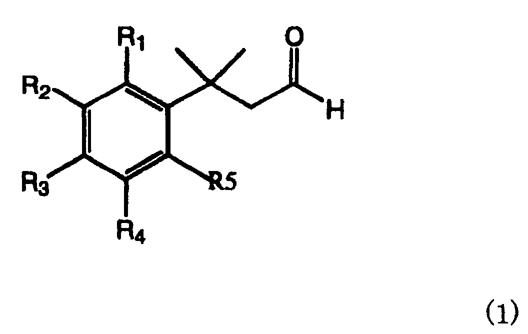

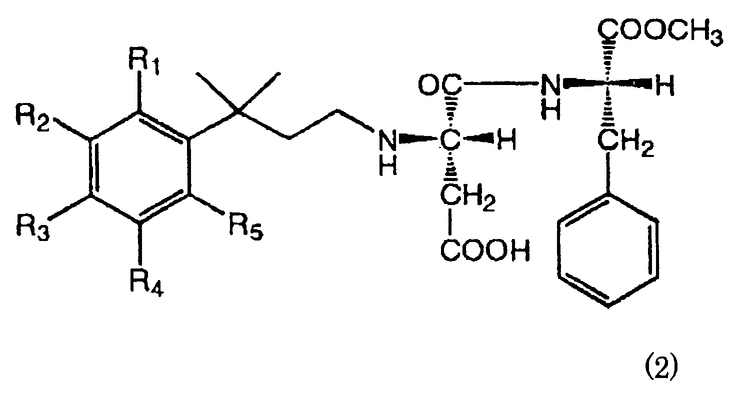

In the above formulae (1) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, a benzyloxy group and a hydroxyalkyloxy group having 2 or 3 carbon atoms, wherein two symbols of R₁ and R₂, or two symbols of R₂ and R₃ may be combined together to denote a methylene dioxy group, and
provided that in the formula (2), any one of R₁, R₂, R₃, R₄ and R₅ does not denote a benzyloxy group.

Further, they have succeeded in synthesizing a novel 3-(3-hydroxy-4-methoxyphenyl)-3-methylbutylaldehyde as an intermediate for the production, and found that this compound is excellent to an extreme as an intermediate for the production of the sweetener having a high degree of sweetness described above.

In addition, they have found a preferable one example in the process for producing efficiently this compound, as shown in the following reaction process 2.

They also have found that 3-(3-hydroxy-4-methoxyphenyl)-3-methylbutylaldehyde is able to become (be) an intermediate for the production of N-[N-[3-(3-hydroxy-4-methoxyphenyl)-3-methylbutyl]-L- α-aspartyl]-L-phenylalanine 1-methyl ester which holds a dominant position industrially as compared to the process for producing 3-(3-benzyloxy-4-methoxyphenyl)-3-methylbutylaldehyde described above. In such case, 3-(3-hydroxy-4-methoxyphenyl)-3-methylbutylaldehyde can be synthesized, for example, in the process for reacting 2-halogenoanisole with 3-methylcrotonic acid, preferably in the presence of an acid;
converting the halogen atom in the 3-(3-halogeno-4-methoxyphenyl)-3-methylbutyric acid thus obtained into a hydroxyl group through an alkaline hydrolysis in the presence of a copper catalyst; and
converting thus obtained carboxylic acid into an aldehyde.

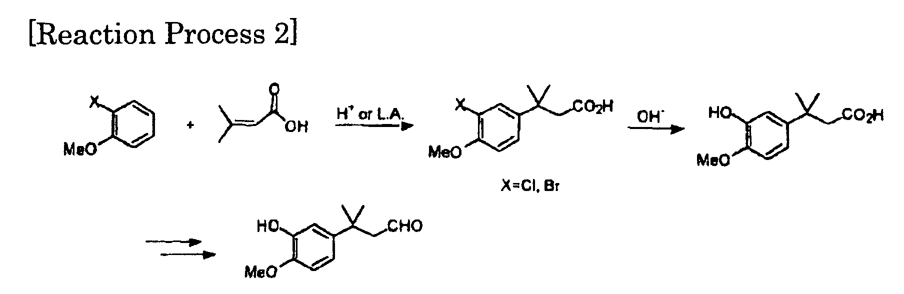

Further, they have studied and investigated a process for producing the object compound of N-[N-[3-(3-hydroxy-4-methoxyphenyl)-3-methylbutyl]-L- α-aspartyl]-L-phenylalanine 1-methyl ester at a high purity, from the object compound containing impurity of moit therein, and also a process for purification thereof including a process for crystallization thereof, and found that the object compound with high purity can be produced conventionally by using a crystallization process, and therefore the object compound can be purified efficiently, and moreover it can be isolated in the crystalline form with high purity, and based on these findings, have reached completion of the present invention.

That is to say, in the present invention the following contents are contained.
[1] A process for producing aspartyl dipeptide ester derivative represented by the following general formula (2), which comprises:
   alkylating reductively aspartame with the aldehyde represented by the following general formula (1):

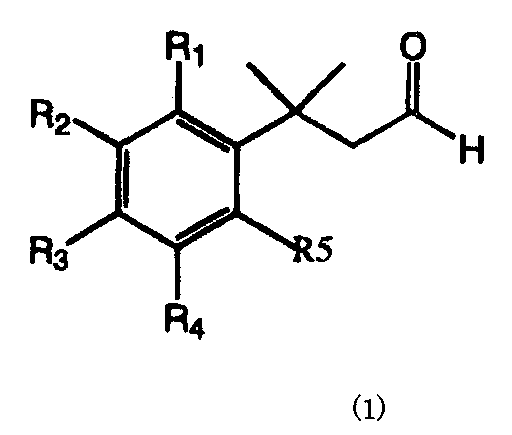

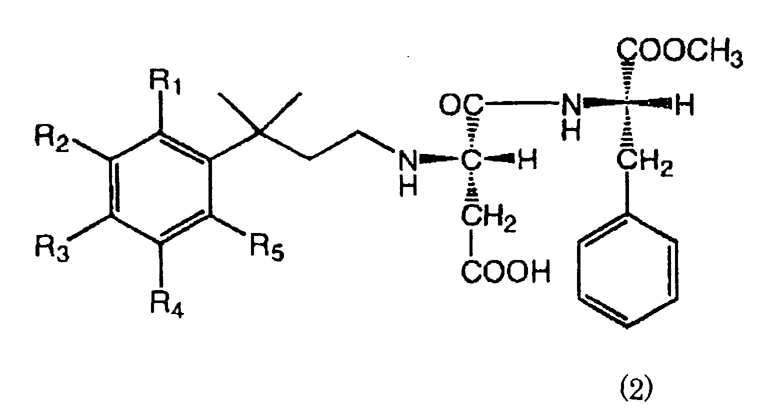

   In the above formulae (1) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, a benzyloxy group and a hydroxyalkyloxy group having 2 or 3 carbon atoms, wherein two symbols of R₁ and R₂, or two symbols of R₂ and R₃ may be combined together to denote a methylene dioxy group, and
   provided that in the formula (2), any one of R₁, R₂, R₃, R₄ and R₅ does not denote a benzyloxy group.
[2] The process as defined above, wherein in the formulae (1) and (2) described above, R₃ is a methoxy group, and R₁, R₂, R₄ and R₅ are a hydrogen atom.
[3] The process as defined above, wherein in the formulae (1) and (2), R₃ is a hydroxyl group, and R₁, R₂, R₄ and R₅ are a hydrogen atom, and in the formula (1), R₃ may be a benzyloxy group.
[4] The process as defined above, wherein in the formulae (1) and (2), R₂ is a methoxy group, R₃ is a hydroxyl group, and R₁, R₄ and R₅ are a hydrogen atom, and in the formula (1), R₃ may be a benzyloxy group.
[5] The process as defined above, wherein in the formulae (1) and (2), R₂ is a hydroxyl group, R₃ is a methoxy group, and R₁, R₄ and R₅ are a hydrogen atom, and in the formula (1), R₂ may be a benzyloxy group.
[6] The process as defined above, wherein in the formulae (1) and (2), R₁ is a hydroxyl group, and R₂, R₃, R₄ and R₅ are a hydrogen atom, and in the formula (1), R₁ may be a benzyloxy group.
[7] The process as defined above, wherein in the formulae (1) and (2), R₁ is a hydroxyl group, R₃ is a methoxy group, and R₂, R₄ and R₅ are a hydrogen atom, and in the formula (1), R₁ may be a benzyloxy group.
[8] The process as defined above, wherein in the formulae (1) and (2), R₁ is a hydroxyl group, R₃ is a methyl group, and R₂, R₄ and R₅ are a hydrogen atom, and in the formula (1), R₁ may be a benzyloxy group.
[9] The process as defined above, wherein in the formulae (1) and (2), R₂ and R₃ are combined together to denote a methylene dioxy group, and R₁, R₄ and R₅ are a hydrogen atom.
[10] The process as defined above, wherein in the formulae (1) and (2), R₂ is a methyl group, R₃ is a methoxy group, and R₁, R₄ and R₅ are a hydrogen atom.
[11] The process as defined above, wherein in the formulae (1) and (2), R₂ is a methyl group, R₃ is a hydroxyl group, and R₁, R₄ and R₅ are a hydrogen atom, and in the formula (1), R₃ may be a benzyloxy group.
[12] The process as defined above, wherein in the formulae (1) and (2), R₂ is a hydroxyl group, R₃ is a methyl group, and R₁, R₄ and R₅ are a hydrogen atom, and in the formula (1), R₂ may be a benzyloxy group.
[13] The process as defined above, wherein the reaction for alkylating reductively said aspartame described above is conducted in the presence of reduction catalyst, and particularly catalyst for hydrogenation.
[14] The process as defined above, wherein said catalyst is palladium carbon or platinum carbon.
[15] The process as defined above, wherein the reaction for alkylating reductively said aspartame described above is conducted in a solvent, and said solvent is an alcohol or a water-containing alcohol.
[16] In the above formulae (1) and (2), R₁, R₂, R₃, R₄ and R₅ are reciprocally independent and each is an any one substituent group selected from the group consisting of a hydrogen atom, a hydroxyl group, a methoxy group, and a methyl group.
   In case that the substituent group is a hydroxyl group, in the formula (1), said substituent group may be a benzyloxy group in place of said hydroxyl group.
   Further, two symbols of R₁ and R₂, or two symbols of R₂ and R₃, respectively may be combined together to be able to form a methylene dioxy group.
[17] A process for producing 3-(3-hydroxy-4-methoxyphenyl)-3-methylbutylaldehyde, which comprises:
   subjecting 3-(3-hydroxy-4-methoxyphenyl)-3-methylbutyric acid to a reaction for converting a carboxyl group into a formyl group.
[18] The process as defined in [17], wherein in said process [17], said 3-(3-hydroxy-4-methoxyphenyl)-3-methylbutyric acid is obtained by any one step of the following steps:
   a. subjecting 3-(3-halogeno-4-methoxyphenyl)-3-methylbutyric acid to a reaction for converting a halogen atom of the substituent group into a hydroxyl group; and
   b. subjecting 3-(3-halogeno-4-methoxyphenyl)-3-methylbutyric acid obtained through the step for reaction of 2-halogenoanisole with 3-methylcrotonic acid,
   to a reaction for converting a halogen atom of the substituent group into a hydroxyl group.
[19] The process as defined in [17], wherein in said process [17], the halogen atom located in said 3-(3-halogeno-4-methoxyphenyl)-3-methylbutyric acid, is a chlorine atom or a bromine atom.
[20] The process as defined in [17], wherein in said process [17] said reaction with 3-methylcrotonic acid is conducted in the presence of an acid.
[21] The process as defined in [17], wherein in said process [17], said reaction for converting a carboxyl group into a formyl group is any one of the following reactions:
   a. a reaction for converting a carboxylic acid to an aldehyde through a half reduction thereof; and
   b. a reaction for converting a carboxyl group into a hydroxymethyl group, and thereafter converting it into a formyl group.
[22] In addition to said reaction [17]∼[21], further a reductive allylation reaction with aspartame is conducted to lead to (produce) N-[N-[3-(3-hydroxy-4-methoxyphenyl)-3-methylbutyl]-L- α-aspartyl]-L-phenylalanine 1-methyl ester.
   Such above process is contained in the present invention.
[23] A process for producing N-[N-[3-(3-hydroxy-4-methoxyphenyl)-3-methylbutyl]-L- α-aspartyl]-L-phenylalanine 1-methyl ester, which comprises:
   subjecting 3-(3-hydroxy-4-methoxyphenyl)-3-methylbutylaldehyde and aspartame to a reductive alkylation reaction.
[24] A benzene derivative represented by the following general formula (3): 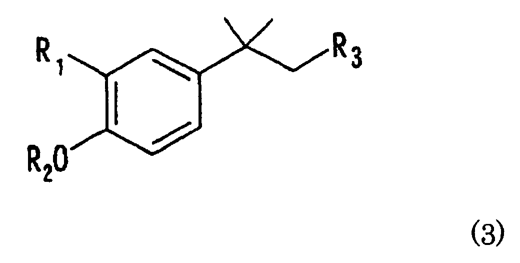
   In the above formula, R₁ denotes any one substituent group of a hydroxyl group, a halogen atom and a lower alkyloxy group having 1 to 4 carbon atoms, R₂ denotes a lower alkyl group having 1 to 4 carbon atoms, and R₃ denotes any one substituent group of a carboxyl group, a formyl group and a hydroxymethyl group,
   provided that the compounds where R₁ is a chlorine atom or a bromine atom, and R₃ is a formyl group, are excluded.
[25] Among the benzene derivatives shown in said general formula (3), a benzene derivative selected from the following compounds a. to e. is particularly desirable:
   a. 3-(3-hydroxy-4-methoxyphenyl)-3-methylbutylaldehyde;
   b. 3-(3-chloro-4-methoxyphenyl)-3-methylbutyric acid;
   c. 3-(3-bromo-4-methoxyphenyl)-3-methylbutyric acid;
   d. 3-(3-hydroxy-4-methoxyphenyl)-3-methylbutyric acid; and
   e. 3-(3-hydroxy-4-methoxyphenyl)-3-methyl-1-butanol.
[26] A process for producing N-[N-[3-(3-hydroxy-4-methoxyphenyl)-3-methylbutyl]-L- α-aspartyl]-L-phenylalanine 1-methyl ester, which comprises:
   subjecting N- [N- [3-(3-hydroxy-4-methoxyphenyl)-3-methylbutyl]-L- α-aspartyl]-L-phenylalanine 1-methyl ester containing impurity to a crystallization step to crystallize said compound.

### Embodiments for carrying out Invention

The embodiments for carrying out the present invention are explained by showing more preferable processes or methods therefor in the followings. However, such embodiments are explained as the more suitable examples for the present invention, and accordingly the present invention is not limited to such contents.

The aspartyl dipeptide ester derivative represented by the following general formula (2) is produced in the process, which comprises:
alkylating reductively aspartame with the aldehyde represented by the following general formula (1):

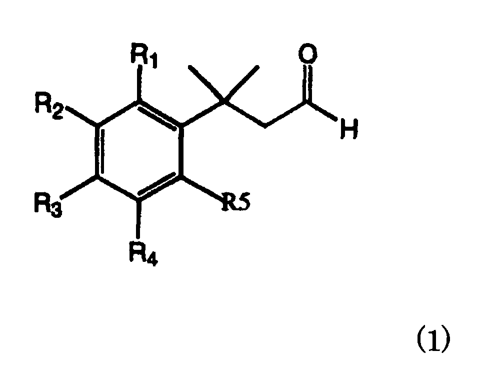

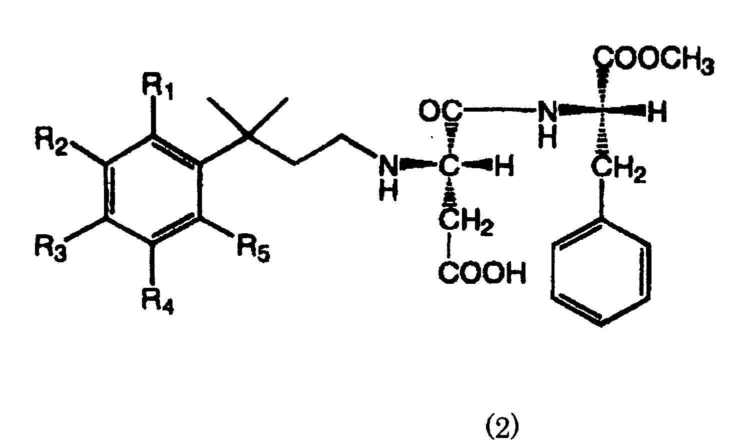

In the reaction for alkylation, it is desirably conducted in a solvent for reaction, and also it is desirably conducted in the presence of reduction catalyst. Particularly, it is reduced desirably with hydrogen by using catalyst usable as reductive alkylation catalyst, for example, catalyst for hydrogenation (hydrogen addition).

In the above formulae (1) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, a benzyloxy group and a hydroxyalkyloxy group having 2 or 3 carbon atoms, wherein two symbols of R₁ and R₂, or two symbols of R₂ and R₃ may be combined together to denote a methylene dioxy group, and
provided that in the formula (2), any one of R₁, R₂, R₃, R₄ and R₅ does not denote a benzyloxy group.

For example, in case that two symbols of R₁ and R₂, or two symbols of R₂ and R₃ may be combined together to denote a methylene dioxy group, the remaining symbols R₃∼R₅, or R₁, R₄ and R₅ are independent each other, and each one has a meaning of the substituent group designated above for the respective each symbol, as a matter of course.

In addition, in the formula (2), any one of R₁, R₂, R₃, R₄ and R₅ does not denote a benzyloxy group. There is a reason therefor in the following. In particular, in case that in the formula (1), any one of R₁, R₂, R₃, R₄ and R₅ denotes a benzyloxy group, when the object compound is produced in the reductive alkylation reaction in the present invention, from a benzyloxy moiety, the benzyl group is removed to convert it into a hydroxyl group.

In the process involved in the present invention, in the solution wherein aspartame has been dissolved or suspended, the aldehyde described above is further dissolved, and then preferably the catalyst is added thereto, and thereafter the reaction may be conducted under hydrogen gas atmosphere. At that time, the reaction proceeds under stirring only. After completion of the reaction, in case that the catalyst is used, the catalyst is removed by filtration, thus obtained filtrate is concentrated to obtain the object compound of aspartyl dipeptide ester derivative as a crude product. Accordingly, from the crude product, the object compound of aspartyl dipeptide ester derivative can be obtained easily through general and ordinary process for purification, such as re-crystallization.

In this respect, as for the production of the aldehyde represented by the general formula (1) described above, as used in the present invention, for example, by making use of the process of the present invention as shown in the reaction process 2 described above, and the processes as shown in the reaction process 1 described above and the reaction process 3 described in the following, it can be produced. For example, according to the reaction process 3 described in the following, a compound having substituent group(s) as objective in the benzene ring is selected as a starting material, and the reaction is conducted, whereby the desired aldehyde can be easily produced. In such case, in case that a hydroxyl group is located in the benzene ring, it is protected as shown in the reaction process 3, as occasion demands, the reaction can be conducted in the same manner as above.

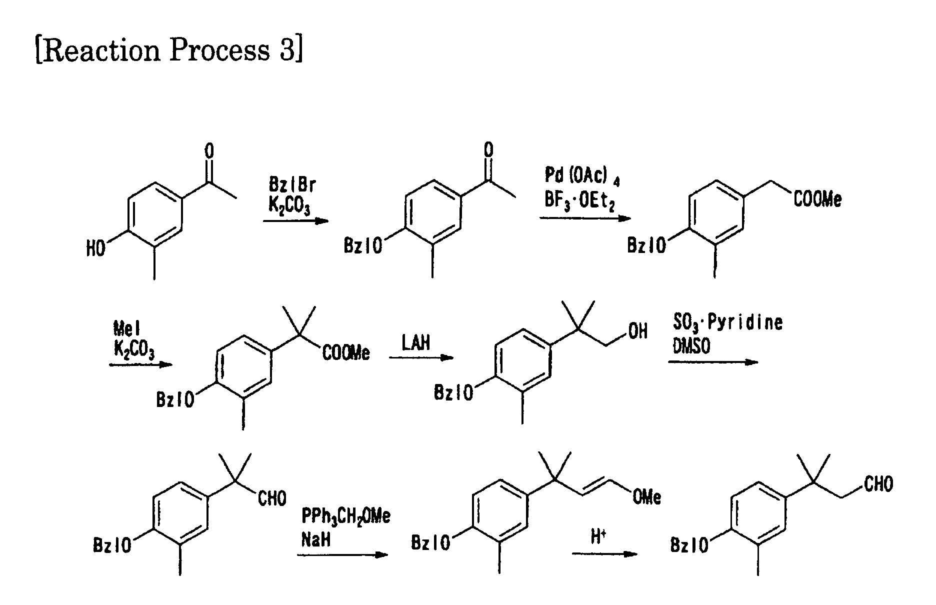

As for the reaction solvent, there is no particular limitations thereto, as far as a material inactive to a ground substance (substrate) for reaction, a catalyst and a product may be employed therefor.
A homogeneous organic solvent which can dissolve aspartame and the aldehyde used in the present invention described above, and which is a single solvent consisted of one kind of organic solvent only or a mixed solvent consisted of plural kinds of organic solvents, or a mixture of such organic solvent with water may be employed therefor.

For the organic solvent, for example, alcohols such as methanol and ethanol, tetrahydrofuran, acetonitrile, dimethylformamide and the like are cited.

Practically, an alcohol such as methanol, or water-containing alcohol such as water-containing methanol is particularly suitable in view of a reaction property.

For the example of the catalyst, if used, a catalyst for hydrogenation, such as palladium, platinum, nickel and rhodium based catalyst, is preferably cited therefor. Specifically, palladium carbon, platinum carbon, rhodium carbon, Raney Nickel and the like are cited therefor. In particular, palladium carbon and platinum carbon are preferable.

The reductive alkylation reaction used in the present invention can be conducted through hydrogenation (hydrogen addition), and in such case, for the hydrogen pressure, preferably 0.1 to 1.0 MPa or so may be selected.

For the reaction temperature, the condition suitable for the reductive alkylation reaction can be selected. In order to suppress (limit) a secondary reaction and to promote the reaction desired (as objective), a temperature range of 15 to 50 °C or so, and a range for reaction time of 2 to 72 hours or so can be preferably selected.

As for a molar ratio of aspartame to the aldehyde as the starting materials used in the present invention, a range of preferably 0.5 to 1.5 moles or so of aspartame per 1 mole of the aldehyde can be used for reaction.

Next, as for the production of 3-(3-hydroxy-4-methoxyphenyl)-3-methylbutylaldehyde, it is explained in the followings based on the reaction process 2 described above as a suitable one example. Since it is explained as a suitable example, the present invention is not limited thereto, as long as a subject matter in the present invention does not come away (is not deviant).

As for the reaction of 2-halogenoanisole, such as 2-chloroanisole, 2-bromoanisole and the like, with 3-methylcrotonic acid, it is conducted without a solvent or in an organic solvent, preferably in the presence of an acid. As for the organic solvent, if employed, there is no special limitations thereto, as far as a material inactive to a ground substance (substrate) for reaction, an acid and a reaction product may be employed therefor. For a preferable solvent, for example, methylene chloride, chloroform, nitrobenzene and the like are cited.

In case that an acid is employed, for the acid to be employed, a proton acid (H⁺), such as sulfuric acid, para (p-)toluenesulfonic acid and hydrogen chloride, a Lewis acid (L.A.), such as aluminum chloride and titanium tetrachloride, and the like, and any acids can be employed. Plural acids can be employed, respectively in the proton acid or Lewis acid, if necessary. Preferably, sulfuric acid, aluminum chloride and titanium tetrachloride are employed. Further, a proton acid may be possibly employed in combination with a Lewis acid, such as combination of hydrogen chloride with aluminum chloride, if necessary. In addition, an acid fixed onto the surface of the solid phase is desirably employed, because a process for treatment may be simplified.

Further, an amount of acid to be employed is not limited particularly. In case that much excess of acid is employed to the 3-methylcrotonic acid, the reaction can be conducted in a shorter time to completion. From the economical point of view, preferably 5 molar equivalents or less, more preferably 3 molar equivalents or less, and further more preferably 0.1 to 3 molar equivalents or so, of the acid to the 3-methylcrotonic acid may be employed.

As for a quantity consumed (employed) of 2-halogenoanisole to the 3-methylcrotonic acid, it is not limited particularly. Preferably 0.5 molar equivalents or more, more preferably 1 molar equivalent or more, and further more preferably 1 to 10 molar equivalents or so, of 2-halogenoanisole to the 3-methylcrotonic acid may be employed.

As for a reaction temperature, there is no particular limitations thereto. The higher the reaction temperature is, the more the secondary reaction proceeds, and at a low temperature, the reaction speed becomes slow to an extreme. Accordingly, a temperature range of, preferably 20 to 180 °C or so, and more preferably 30 to 100 °C or so is employed.

As described above, in case that 3-(3-hydroxy-4-methoxyphenyl)-3-methylbutyric acid is produced from the 3-(3-halogeno-4-methoxyphenyl)-3-methylbutyric acid obtained in the first stage, a reaction for converting the halogen atom substituted at the 3-position of the phenyl group into a hydroxyl group may be employed. For example, by heating 3-(3-halogeno-4-methoxyphenyl)-3-methylbutyric acid in the presence of a copper catalyst in the alkaline aqueous solution, the halogen atom can be easily converted into a hydroxyl group.

In such case, as regards the alkaline material employed, a metal hydroxide, such as sodium hydroxide, potassium hydroxide and the like can be put into operation industrially at a low cost, and therefore it is preferable. Further, as for an amount used of the alkaline material, there is no particular limitations thereto. For example, it is sufficient that 1 to 10 moles or so thereof to 1 mole of 3-(3-halogeno-4-methoxyphenyl)-3-methylbutyric acid is employed.

To a reaction temperature when converting it to the hydroxyl group, there is no particular limitations. The higher the reaction temperature is, the more the secondary reaction proceeds, and at a low temperature, the reaction speed becomes slow to an extreme. Accordingly, a temperature range of, preferably 100 to 250 °C or so, and more preferably 150 to 200 °C or so is employed.

As for a kind of the copper catalyst when it is employed in the above reaction, it is not particularly limited, as long as the catalyst as employed therein may release univalent or divalent copper ion in the aqueous solution. For example, copper oxide(I), copper oxide(II), sulphate of copper(II), and the like are cited therefor. Preferably, sulphate of copper(II) is employed.

In order to produce the aldehyde from 3-(3-hydroxy-4-methoxyphenyl)-3-methylbutyric acid obtained in the above reaction, the carboxylic acid may be made to a half reduction thereof. Preferably the compound can be reduced directly by half for a half reduction thereof, based on the process described in Chemistry Letters, pp. 1143-1144, published in 1998, November, to be converted into 3-(3-hydroxy-4-methoxyphenyl)-3-methylbutylaldehyde as objective. This process is a process for reducing 3-(3-hydroxy-4-methoxyphenyl)-3-methylbutyric acid with hydrogen in the organic solvent, under addition of pivalic acid anhydride, palladium acetate and triphenylphosphine derivative.

As for the organic solvent, there is no particular limitations thereto, as far as a material inactive to a ground substance (substrate) for reaction, a catalyst and a product may be employed therefor. For example, acetone, tetrahydrofuran, toluene and the like are preferably employed. As for an amount employed of the pivalic acid anhydride, equimolecular or more of the compound to 3-(3-hydroxy-4-methoxyphenyl)-3-methylbutyric acid, may be employed. For example, 1 to 5 moles or so of the compound to 1 mole of 3-(3-hydroxy-4-methoxyphenyl)-3-methylbutyric acid may be preferably employed. As triphenylphosphine derivative, triphenylphosphine and tritolylphosphine may be preferably employed. The palladium acetate and triphenylphosphine derivative are used as a catalyst.
As for the quantities consumed thereof, in case of palladium acetate, preferably 0.1 to 5 moles % and above all preferably 0.5 to 3 mole % thereof to the ground substance (substrate) can be employed. And, in case of triphenylphosphine derivative, preferably 5 times mole % or more and more preferably 5 to 7 times mole % thereof to the palladium acetate can be employed. As for the reaction temperature employed in the reaction, it is not limited particularly. For, example, the reaction is conducted preferably at 40 to 100 °C, and above all preferably at 60 to 80 °C. At a higher temperature, the reaction is promoted, and thus can be completed and finished in a short time. Accordingly, at a comparatively high temperature, it can be conducted at an advantage.

On the other hand, in place of the production of aldehyde through a half reduction from the carboxylic acid, as an another process, the carboxyl group therein is reduced completely to a hydroxymethyl group, and thereafter oxidized partially to be able to produce the above aldehyde derivative as objective. In this case, the above object compound can be easily produced, with the use of reduction- partial oxidation, which is known in itself (Journal of Organic Chemistry, vol. 48, No. 25, 5043-5048, 1983).

For example, through the above half reduction or the above reduction from 3-(3-halogeno-4-methoxyphenyl)-3-methylbutyric acid, respectively in the same manner, 3-(3-halogeno-4-methoxyphenyl)-3-methylbutylaldehyde, or 3-(3-halogeno-4-methoxyphenyl)-3-methyl-1-butanol can be also produced.

In the production of N-[N-[3-(3-hydroxy-4-methoxyphenyl)-3-methylbutyl]-L-α-aspartyl]-L-phenylalanine 1-methyl ester from 3-(3-hydroxy-4-methoxyphenyl)-3-methylbutylaldehyde thus obtained, there is no particular difficulty. It can be easily produced by the step of a reductive alkylation of the aldehyde with α-L-aspartyl-L-phenylalanine methyl ester (aspartame) under condition for hydrogenation. Specifically, in the solvent which can dissolve the starting materials, for example, alcohol, water-containing alcohol, or the like, in the presence of the catalyst for a reductive alkyaltion, for example, a catalyst such as palladium based catalyst, a reductive alkylation reaction is conducted with hydrogen, more preferably, under suitable or effective reaction temperature and pressure to be able to produce the above object compound.

As for the reaction solvent, there is no particular limitations thereto, as far as a material inactive to a ground substance (substrate) for reaction, a catalyst and a product may be employed therefor.

A homogeneous organic solvent which can dissolve aspartame and the aldehyde described above, and which is a single solvent consisted of one kind of organic solvent only or a mixed solvent consisted of plural kinds of organic solvents, or a mixture of such organic solvent with water may be employed therefor.

For the organic solvent, for example, alcohols such as methanol and ethanol, tetrahydrofuran, acetonitrile, dimethylformamide and the like are cited.

Practically, an alcohol such as methanol, or water-containing alcohol such as water-containing methanol is particularly suitable in view of a reaction property.

For the example of the catalyst, if used, a catalyst for hydrogenation, such as palladium, platinum, nickel and rhodium based catalyst, is preferably cited therefor.

The reductive alkylation reaction used in the present invention can be conducted through hydrogenation (hydrogen addition), and in such case, for the hydrogen pressure, preferably 0.1 to 1.0 MPa or so may be selected.

For the reaction temperature, the condition suitable for the reductive alkylation reaction can be selected. In order to suppress (limit) a secondary reaction and to promote the reaction desired (as objective), a temperature range of 15 to 50 °C or so, and a range for reaction time of 2 to 72 hours or so can be preferably selected.

As for a molar ratio of aspartame to the aldehyde as the starting materials used in the present invention, a range of preferably 0.5 to 1.5 moles or so of aspartame per 1 mole of the aldehyde can be used for reaction.

N-[N-[3-(3-hydroxy-4-methoxyphenyl)-3-methylbutyl]-L-α-aspartyl]-L-phenylalanine 1-methyl ester thus obtained as a material to be purified, is subjected to a step for crystallization (crystallization step), and the said compound is made to crystallization to remove various impurity(ies) present inside the system of reaction described above, whereby N-[N-[3-(3-hydroxy-4-methoxyphenyl)-3-methylbutyl]-L-α-aspartyl]-L-phenylalanine 1-methyl ester with high purity can be produced.

As the N-[N-[3-(3-hydroxy-4-methoxyphenyl)-3-methylbutyl]-L-α-aspartyl]-L-phenylalanine 1-methyl ester containing impurity, a solution, a solid or an intermediate (intermediary body) therebetween such as a slurry, containing the object compound, and the like can be cited.

In the object compound described above, mostly at least one compound of aspartame, aspartame derivative, peptide derivative, amino acid, amino acid derivative, aldehyde and its derivative may be contained as impurity of moit. The present invention is suitable for purifying the object compound described above containing such impurity (a material to be purified). The state of the material to be purified includes a solution form, a solid form, or a form of an intermediate stage therebetween as the case may be, and the like, as described above. As long as the object compound contains the impurity, there is no particular limitations to how to contain the impurity therein.

As one characteristic matter involved in the process of the present invention, independent to the state of the object compound described above, from the material containing impurity to be purified, the object compound is made to crystallization with the use of a suitable solvent for crystallization, whereby the object compound with high purity is produced, and the crystals thereof are separated to purify the same.

In order to obtain the object compound of N-[N-[3-(3-hydroxy-4-methoxyphenyl)-3-methylbutyl]-L-α-aspartyl]-L-phenylalanine 1-methyl ester as the solid form, for example, it is thought that the reaction solution thereof is purified with a silica gel column chromatography and the thus obtained fractions of the object compound extracted is concentrated to solidification, for one process to obtain the same. However, this process is high in cost, and problematic in treatment of waste (used) silica gel or the like, and therefore is undesirable for industrial operation. Further, in the powder X-ray analysis obtained by the present inventors, the solid obtained in this process is in the amorphous state, and is also undesirable in stability.

The object compound described above is useful as a sweetener used for a food, a pharmaceutical product and the like, and it is requested that the compound is a final form at a high purity and excellent in stability.

As a typical example of the material to be purified, the reaction solution containing the object compound obtained on conducting the reductive alkylation reaction to produce the object compound with the use of aspartame and 3-(3-hydroxy-4-methoxyphenyl)-3-methylbutylaldehyde or its derivative (said derivative wherein the hydroxyl group is protected, and the like) can be cited. As for such material to be purified in the liquid state, in case that the solution wherein insoluble material undesirable for the crystallization step, for example, the used catalyst or the like is present, is purified, the insoluble material is removed previously (in advance) by separation with filtration. The thus obtained solution is subjected to the crystallization under a condition suitable for the crystallization of the object compound to crystallize the object compound, and as a result, the compound can be separated for purification.

For example, in case that the solution described above contains the solvent for the crystallization solvent of the object compound, as a process for purification thereof the solution is concentrated with concentration operation under reduced pressure or the like until to a condition desirable for the crystallization of the object compound, or after such concentration, among the crystallization solvents for the object compound a suitable crystallization solvent is specifically selected and is added secondarily to thus concentrated solution until to a condition desirable for the crystallization of the object compound, and the thus obtained solution is cooled or concentrated where necessary, and thereafter, cooled until to a condition suitable for the crystallization, or the like to be able to crystallize the object compound.

On the other hand, for example, in case that the solution described above uses a solvent undesirable for the crystallization of the object compound, as a process for purification thereof the solution is concentrated with a concentration operation under reduced pressure or the like to remove the solvent completely by distillation to solidification, and thereafter, among the crystallization solvents for the object compound a suitable crystallization solvent is specifically selected, and the crystallization step is conducted in the same manner as above, to be able to crystallize the object compound. As a matter of course, the process can be applied to the case where the material to be purified does not contain a solvent. Further, independent to the fact whether the solution described above contains a crystallization solvent for the object compound or not, a process for substitution of solvent is also as effective means therefor.

In the present invention, in case that the material to be purified contains an nonpolar solvent such as aldehyde and its derivative, as the impurity of moit, as described above, by further combining a step for extraction with solvent therewith, before the crystallization step, during the crystallization step or after the crystallization step, for conducting the crystallization process, the crystals of the object compound with higher purity can also be produced. According to such extraction with solvent, a process for removing impurity is conducted preferably in the state where the crystals are dissolved therein. However, it may be conducted in the state where the crystals are not dissolved therein completely, that is in the slurry state. For example, in case that the solution such as the above synthetic reaction solution is subjected to the process for extraction with solvent, the purification can be conducted in the following conventional means. To the reaction solution directly or to the solution which has been concentrated partially where necessary, a suitable solvent for extraction and water where necessary are added to dissolve the impurity in the solvent in the state of solution and thereby to remove it from the aqueous layer. An aqueous layer at the time of such extraction with water, may contain, for example, at least one solvent selected from the group consisting of methanol, ethanol, isopropyl alcohol, tetrahydrofuran, acetonitrile, acetic acid and ethyl acetate, which is a solvent usable for the reductive alkylation reaction, as far as it does not work against the separating nature of the solvent at the time of extraction.

The solvent used for the extraction with solvent in the present invention is a solvent which can not be mixed with water homogeneously. For example, at least one solvent selected from toluene, diethyl ether, chloroform, dichloromethane, hexane, ethyl acetate, propyl acetate, isopropyl acetate and butyl acetate, can be employed preferably therefor. More preferably, toluene may be employed, in view of operability.

For the solvent used for the crystallization step in the present invention, at least one solvent selected from a lower alcohol such as methanol, ethanol, isopropyl alcohol and the like, ketones such as acetone, methyl ethyl ketone, methyl isobutyl ketone and the like, esters such as methyl acetate, ethyl acetate, propyl acetate, isopropyl acetate, butyl acetate and the like, tetrahydrofuran, acetonitrile and toluene, or a mixed solvent of at least one solvent of these organic solvents with water, are preferably employed. More preferably, methanol, ethanol, acetone and water can be employed. Further, preferably, methanol, ethanol and/or acetone which are good solvents can be employed properly in combination with water which is a poor solvent.

To a process for crystallization, there is no particular limitations. In order to solve the problem before the present invention more easily, for example, a process obtained by combining properly a partial or perfect removal of the solvent through concentration, addition of the solvent for crystallization described above and crystallization under cooling can be suitably conducted. For example, in case that the material to be purified is a synthetic reaction solution containing said N-[N-[3-(3-hydroxy-4-methoxyphenyl)-3-methylbutyl]-L-α-aspartyl]-L-phenylalanine 1-methyl ester obtained in the process for alkylating reductively aspartame and 3-(3-hydroxy-4-methoxyphenyl)-3-methylbutylaldehyde in a mixed solvent of methanol and water (mixing ratio of 3 : 2 v/v), the object compound in the crystalline form at a high purity can be obtained in the following extremely conventional process. That is, from the solution the catalyst is separated by filtration, toluene is added thereto for extraction with solvent, and thereby the impurity is removed, and thus obtained aqueous layer is concentrated under reduced pressure to remove methanol partially, and cooled to crystallize the object compound for separation of the crystals.

To a process for separation of the crystals, there is no particular limitations. For example, a usual method such as separation by filtration, centrifugal separation and the like can be employed. Further, after separation of the crystals, the crystals can be dried where necessary, and to this process for drying, there is also no particular limitations. For example, a usual method such as drying under reduced pressure, throughflow drying, and the like can be employed.

For a crystallizing condition suitable for crystallization of the object compound in the present invention, because it is varied according to, for example, kind of crystallization solvent, composition of solvent, quantity employed of solvent, method of crystallization, and the like, or a combination thereof, uniformly explanations can not be given. For example, in case that the present invention is conducted with crystallization under cooling, mostly, it may be conducted in the following range shown. That is, for a concentration of the object compound in the solution to be subjected to a crystallization process is 0.5 to 20 g/dl or so, preferably 1 to 15 g/dl or so, and more preferably 2 to 10 g/dl or so
may be preferably employed. When the concentration is lower too much, yield for crystallization is lowered, and when it is higher too much, purity is lowered, and therefore they are not desirable. To a temperature for starting crystallization there is no particular limitations. For example, 40 to 80 °C or so, and preferably 50 to 70 °C or so may be preferably selected. When the temperature is higher too much, a problem such as decomposition of the object compound, distillation (vaporization) of the crystallization solvent, or the like is raised, and therefore it may be undesirable. To a temperature for finishing crystallization, there is no particular limitations. It may be conducted at a temperature where the solution is not solidified. It may be cooled until to preferably 20 to 5 °C or so, and more preferably 15 to 5 °C or so. To a cooling speed, there is no particular limitations.

In the present invention, the crystals of the object compound obtained in the process for purification described above, are contained. The crystals of the object compound exhibit the following physical properties:

For the N-[N-[3-(3-hydroxy-4-methoxyphenyl) -3-methylbutyl]-L-α-aspartyl]-L-phenylalanine 1-methyl ester in the crystalline form, it exhibits peaks of diffractive X-ray in at least diffraction angles of 8.3° , 19.5° and 21.2° (2θ, CuKα ray ) when determined in the powder X-ray diffractometry.

Further, in the present invention, a sweetener, or a food and drink or other product with a sweet taste imparted, comprising the crystal at a high purity, as described above as an effective ingredient, are contained. In this case, it may further comprise a carrier or a bulking agent for a sweetener.

In addition, a process for imparting a sweet taste, which comprises:
adding the crystals as described above, or including said crystals in a product such as a food and drink or the like in need of a sweet taste imparted or the intermediate product therefor during manufacture, is also contained in the present invention.

In case that a sweetener is produced, the product such as a food and drink, or the like (including a pharmaceutical product, and the like) having a sweet taste imparted, is produced, or a sweet taste is imparted into a product in need of a sweet taste or the intermediate product therefor, for example, the same during manufacture, it can come to operation by making use of known methods or the like so far, as a process for producing a sweetener with the use of a sweetening component, or as a process for imparting a sweet taste.

In case that the crystals obtained in the present invention, are used for sweeteners or for foods and drinks (beverages), in order to give stability much more thereto as described above, a carrier, a thickening agent (viscosity improver), a bulking agent, and/or an excipient (diluting agent) for sweeteners, where necessary, can be employed. In such case, for example, a thickening agent (viscosity improver), a bulking agent, an excipient (diluting agent), and the like for sweeteners known or used, so far can be employed.

For such carrier or the like, a saccharide which can be also used as a stabilizing agent (material), or the like can be also employed. Except for such material, the material which has been used so far or could be used in the past, as a carrier for a sweetener can also be employed.

The object compound can also be produced in the form of a sweetener, and further, except for the form of a sweetener, by using a component necessary for a food and drink and an excipient, it can be taken effect in the form of a food and drink, such as a frozen dessert or the like.

In addition, as a sweetener for a product, such as a food and drink or the like in need of a sweet taste imparted, for example, confectionary (frozen dessert, jelly, cake, candy (sweet or the like), bread, chewing gums, hygiene product, cosmetics (including an oral composition such as dentifrice and others), a pharmaceutical product, and various veterinary product for animal other than for humans or the like, the object compound can be also employed. The crystals obtained in the present invention can be used also in the form of the product (article of manufacture) with a sweet taste imparted thus above, and also in the process for imparting a sweet taste to said product in need of a sweet taste to be imparted.

### Brief Description of the Drawing

### [Figure 1]

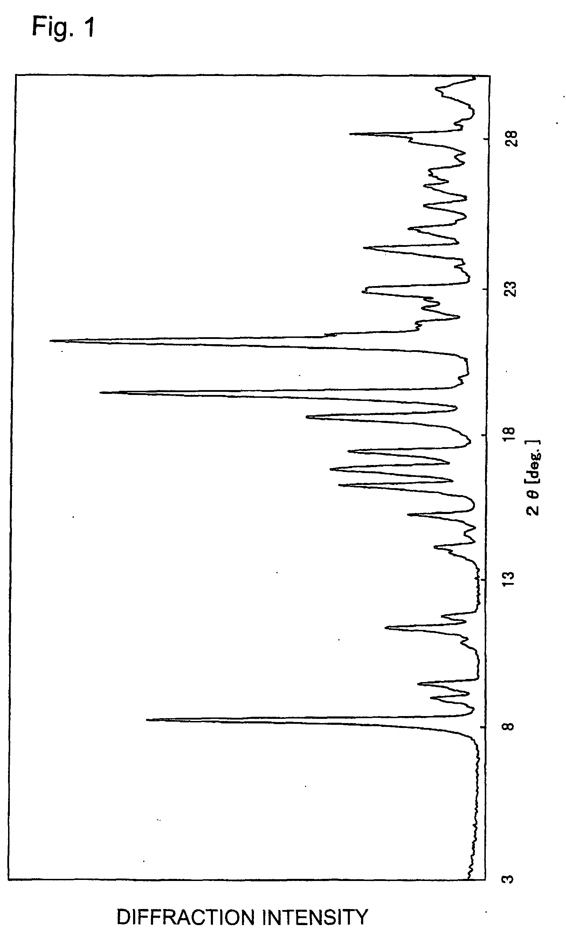

Figure 1 is a powder X-ray diffraction pattern of N-[N-[3-(3-hydroxy-4-methoxyphenyl)-3-methylbutyl]-L-α-aspartyl]-L-phenylalanine 1-methyl ester in the crystalline form obtained in the present invention.

### Examples

The present invention will be explained further in detail with reference to the following Examples therefor.

### (Example 1)

### Synthesis of 3-(3-chloro-4-methoxyphenyl)-3-methylbutyric acid

To 2-chloroanisole (100.0 g), 95 % sulfuric acid (72.4 g) and 3-methylcrotonic acid (35.1 g) were added and the mixture was stirred under heating at 70 °C for 67 hours, and thereafter the reaction was stopped by addition of water (200 ml) thereto. Thus obtained mixture was extracted with methylene chloride (400 ml), and to the separated organic layer 1 normal (1N) caustic soda aqueous solution (200 ml) was added for further extraction. To the separated aqueous layer hydrochloric acid (HCl) was added to make it acidification, and the mixture was extracted with methylene chloride and the solvent therein was distilled off. Thus obtained residue was re-crystallized with ethyl acetate and hexane to obtain 3-(3-chloro-4-methoxyphenyl)-3-methylbutyric acid (10.9 g, yield of 12.7 % to 3-methylcrotonic acid).
¹H NMR (CDCl₃) δ : 1. 4 3 (s, 6H), 2.61 (s, 2H), 3.87 (s, 3H), 6.86 (d, J=8.6 Hz, 1H), 7.10 - 7.23 (m, 1H), 7.35 (d, J=2.4 Hz, 1H). ESI-MS;
Calculation: C₁₂H₁₅³⁵ClO₃=242.3, Analysis: 241.3(MH⁻).

### (Example 2)

### Synthesis of 3-(3-bromo-4-methoxyphenyl)-3-methylbutyric acid

To 2-bromoanisole (50 g), 95 % sulfuric acid (27.6 g) and 3-methylcrotonic acid (5.35 g) were added and the mixture was stirred under heating at 70 °C for 27 hours, and thereafter the reaction was stopped by addition of water (100 ml) thereto. Thus obtained mixture was extracted with methylene chloride (100 ml), and to the separated organic layer 1 normal (1N) caustic soda aqueous solution (100 ml) was added for further extraction. To the separated aqueous layer hydrochloric acid (HCl) was added to make it acidification, and the mixture was extracted with methylene chloride and the solvent therein was distilled off. Thus obtained residue was re-crystallized with ethyl acetate and hexane to obtain 3-(3-bromo-4-methoxyphenyl)-3-methylbutyric acid (3.1 g, yield of 20.3 % to 3-methylcrotonic acid).

### (Example 3)

### Synthesis of 3-(3-bromo-4-methoxyphenyl)-3-methylbutyric acid

A mixture of 2-bromoanisole (102.4 g) and 3-methylcrotonic acid (16.1 g) was stirred for mixing, and aluminum chloride (23.5 g) was added thereto. The mixture was stirred under heating at 70 °C for 5 hours, and thereafter the reaction solution was cooled to a room temperature. After that, the reaction was stopped by addition of 6 normal (6N) hydrochloric acid (300 ml) thereto. Thus obtained mixture was extracted with toluene (300 ml), and the separated organic layer was further extracted with 1 normal (1N) caustic soda aqueous solution (500 ml). Subsequently, to the separated aqueous layer 6N-hydrochloric acid (HCl) was added to make it acidification, and the mixture was extracted with toluene (600 ml) and the organic layer was concentrated under reduced pressure to obtain crude crystals thereof. Thus obtained crude crystals were re-crystallized with ethyl acetate and hexane to obtain 3-(3-bromo-4-methoxyphenyl)-3-methylbutyric acid (20.7 g, yield of 45 % to 3-methylcrotonic acid).
¹HNMR (CDCl₃) δ : 1. 4 3 (s, 6H), 2.61 (s, 2H), 3.87 (s, 3H), 6.84 (d, J=8.7 Hz, 1H), 7.20 - 7.29 (m, 1H), 7.52 (d, J=2.4 Hz, 1H). ESI-MS;
Calculation: C₁₂H₁₅⁷⁹BrO₃=286.2, Analysis: 285.2(MH⁻).

### (Example 4)

### Synthesis of 3-(3-hydroxy-4-methoxyphenyl)-3-methylbutyric acid

Into a stainless steel reaction vessel withstand pressure, 3-(3-bromo-4-methoxyphenyl)-3-methylbutyric acid (18.8 g), cupric sulfate 5 hydrate (8.2 g), sodium hydroxide (48.16 g) and distilled water (159 ml) in these order were filled, and the mixture was stirred for 1 hour at room temperature and then for 10 hours under heating at 160 °C, and then cooled to a room temperature.
To thus obtained reaction solution, hydrochloric acid (HCl) was added to make it acidification, and the mixture was extracted with ethyl acetate and the organic layer was washed with sodium chloride saturated aqueous solution. The solvent therein was distilled off under reduced pressure to obtain crude crystals. Thus obtained crude crystals were re-crystallized with ethyl acetate and hexane to obtain 3-(3-hydroxy-4-methoxyphenyl)-3-methylbutyric acid (10.5 g, yield of 72 %).
¹HNMR (CDCl₃ δ : 1 . 4 2 (s, 6H), 2.60 (s, 2H), 3.86 (s, 3H), 6.78 (d, J=8.5 Hz, 1H), 6.84 (dd, J=2.2, 8.5 Hz, 1H), 6.95 (d, J=2.2 Hz, 1H).
ESI-MS;
Calculation: C₁₂H₁₆O₄=224.3, Analysis: 223.2(MH⁻).

### (Example 5)

### Synthesis of 3-(3-hydroxy-4-methoxyphenyl)-3-methylbutyl aldehyde

Into a chemical reactor for hydrogen addition (hydrogenation) under elevated pressure, 3-(3-hydroxy-4-methoxyphenyl)-3-methylbutyric acid (13.6 g), pivalic acid anhydride (22.8 g) and acetone (100 ml) were filled, and thereafter the mixture was bubbled with nitrogen gas for 30 minutes to substitute nitrogen gas completely for the gas in the system of reaction, whereby the system was filled with nitrogen gas. Subsequently, palladium acetate (137 mg) produced previously and tri-p-tolylphosphine (930 mg) in tetrahydrofuran solution (50 ml) were added thereto, and the mixture was stirred under hydrogen pressure of 5 MPa at 80 °C for 24 hours for reaction.

From the reaction solution, acetone was distilled off, and thus obtained residue was purified by the use of column chromatography to obtain 3-(3-hydroxy-4-methoxyphenyl)-3-methylbutylaldehyde (10.2 g, yield: 80 %).
¹HNMR (CDCl₃) δ : 1.41 (s, 6H), 2.61 (d, J=3.0 Hz, 2H), 3.87 (s, 3H), 6.72 - 6.84 (m, 2H), 6.98 (d, J=1.9 Hz, 1H), 9.49 (t, J=3.0 Hz, 1H).
ESI-MS;
Calculation: C₁₂H₁₆O₃=208.3, Analysis: 207.2(MH⁻).

### (Example 6)

### Synthesis of N-[N-[3-(3-hydroxy-4-methoxyphenyl)-3-methylbutyl]-L-α-aspartyl]-L-phenylalanine 1-methyl ester

Aspartame (5.89 g, 20.0 mmol) and 3-(3-hydroxy-4-methoxyphenyl)-3-methylbutylaldehyde (3.96 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours. Thus obtained reaction solution was filtrated to remove the catalyst, and the filtrate was subjected to the high performance liquid chromatography (HPLC) for determination and thereby the title compound (5.38 g, 10.7 mmol, 56.6 %) was produced.

### (Example 7)

### Production of N-[N-[3-(4-methoxyphenyl)-3-methylbutyl]-L-α-aspartyl]-L-phenylalanine 1-methyl ester

Aspartame (5.89 g, 20.0 mmol) and 3-(4-methoxyphenyl)-3-methylbutylaldehyde (3.65 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the high performance liquid chromatography (HPLC) for determination and thereby the title compound (6.45 g, 13.7 mmol, 72.2 %) was produced.

### (Example 8)

### Production of N-[N-[3-(4-hydroxyphenyl)-3-methylbutyl]-L-α-aspartyl]-L-phenylalanine 1-methyl ester

Aspartame (5.89 g, 20.0 mmol) and 3-(4-hydroxyphenyl)-3-methylbutylaldehyde (3.38 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (5.59 g, 12.3 mmol, 64.5 %) was produced.

### (Example 9)

### Production of N-[N-[3-(3-methoxy-4-hydroxyphenyl)-3-methylbutyl]-L-α-aspartyl]-L-phenylalanine 1-methyl ester

Aspartame (5.89 g, 20.0 mmol) and 3-(3-methoxy-4-hydroxyphenyl)-3-methylbutylaldehyde (3.96 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (5.74 g, 11.8 mmol, 62.2 %) was produced.

### (Example 10)

### Production of N-[N-[3-(2-hydroxyphenyl)-3-methylbutyl]-L-α-aspartyl]-L-phenylalanine 1-methyl ester

Aspartame (5.89 g, 20.0 mmol) and 3-(2-hydroxyphenyl)-3-methylbutylaldehyde (3.39 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (5.81 g, 12.3 mmol, 64.5 %) was produced.

### (Example 11)

### Production of N-[N-[3-(2-hydroxy-4-methoxyphenyl)-3-methylbutyl]-L-α-aspartyl]-L-phenylalanine 1-methyl ester

Aspartame (5.89 g, 20.0 mmol) and 3-(2-hydroxy-4-methoxyphenyl)-3-methylbutylaldehyde (3.96 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (4.21 g, 8.38 mmol, 44.1 %) was produced.

### (Example 12)

### Production of N-[N-[3-(2-hydroxy-4-methylphenyl)-3-methylbutyl]-L-α-aspartyl]-L-phenylalanine 1-methyl ester

Aspartame (5.89 g, 20.0 mmol) and 3-(2-hydroxy-4-methylphenyl)-3-methylbutylaldehyde (3.65 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (4.17 g, 8.57 mmol, 45.1 %) was produced.

### (Example 13)

### Production of N-[N-[3-(3,4-methylenedioxyphenyl)-3-methylbutyl]-L-α-aspartyl]-L-phenylalanine 1-methyl ester

Aspartame (5.89 g, 20.0 mmol) and 3-(3,4-methylenedioxyphenyl)-3-methylbutylaldehyde (3.92 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (6.4 g, 13.2 mmol, 69.7 %) was produced.

### (Example 14)

### Production of N-[N-[3-(3-methyl-4-methoxyphenyl)-3-methylbutyl]-L-α-aspartyl]-L-phenylalanine 1-methyl ester

Aspartame (5.89 g, 20.0 mmol) and 3-(3-methyl-4-methoxyphenyl)-3-methylbutylaldehyde (3.92 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (6.06 g, 12.5 mmol, 66.0 %) was produced.

### (Example 15)

### Production of N-[N-[3-(3-methyl-4-hydroxyphenyl)-3-methylbutyl]-L-α-aspartyl]-L-phenylalanine 1-methyl ester

Aspartame (5.89 g, 20.0 mmol) and 3-(3-methyl-4-hydroxyphenyl)-3-methylbutylaldehyde (3.65 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (5.65 g, 12.0 mmol, 63.2 %) was produced.

### (Example 16)

### Production of N-[N-[3-(3-hydroxy-4-methylphenyl)-3-methylbutyl]-L-α-aspartyl]-L-phenylalanine 1-methyl ester

Aspartame (5.89 g, 20.0 mmol) and 3-(3-hydroxy-4-methylphenyl)-3-methylbutylaldehyde (3.65 g, 19.0 mmol) were added to 80 % methanol aqueous solution (200 ml), and the mixture was stirred at 40 °C in a short time. To this solution, 10 % palladium carbon in the water content of 50 % (1.78 g) was added, and the mixture was stirred under hydrogen atmosphere of normal pressure (0.1 MPa) at 40 °C for 40 hours for reaction. Thus obtained reaction solution was filtrated to remove the catalyst, and the filtrate was subjected to the HPLC for determination and thereby the title compound (5.84 g, 12.4 mmol, 65.5 %) was produced.

### (Example 17)

### Synthesis of N-[N-[3-(3-hydroxy-4-methoxyphenyl)-3-methylbutyl]-L-α-aspartyl]-L-phenylalanine 1-methyl ester, Crystallization thereof, and Separation of the crystals thereof

3-(3-Hydroxy-4-methoxyphenyl)-3-methylbutylaldehyde (6.677 g, 25.2 mmol) was dissolved in 80 % methanol aqueous solution (272 ml), and aspartame (8.446 g, 27.8 mmol) was added thereto to prepare a slurry solution. 10 % Palladium carbon in the water content of 50 % (2.86 g) was added thereto under nitrogen stream, and thereafter substitution of hydrogen for the reaction system was performed and the mixture as it is was stirred at 25 °C for 24 hours. After substitution of nitrogen therefor, the catalyst was separated by filtration, and further washed with methanol (30 ml). After that, to the filtrate water (193 ml) was added, and thus obtained mixture was extracted with toluene (247.6 ml) twice. The separated methanol/water layer was concentrated under reduced pressure to approximately one second (1/2) of quantity thereof by weight. After that, the concentrated solution was cooled sequentially from 75 °C to 5 °C to precipitate crystals. Thus separated crystals were dissolved in 50 % methanol aqueous solution (260 ml) at 75 °C, and thus obtained solution was cooled to 5 °C to precipitate crystals. The crystals were dried under reduced pressure to obtain N-[N-[3-(3-hydroxy-4-methoxyphenyl)-3-methylbutyl]-L-α-aspartyl]-L-phenylalanine 1-methyl ester (8.46 g, 17.1 mmol, yield of 67.6 % based on the aldehyde) in the white crystalline form.

(The purity in the HPLC analysis was not less than 98 %.)
¹H NMR (DMSO-d₆) δ : 1.14 (brs, 6H), 1.54 - 1.68 (m, 2H), 2.04 - 2.22 (m, 3H), 2.24 - 2.34 (dd, 1H), 2.84 - 2.94 (dd, 1H), 3.00 - 3.08 (dd, 1H), 3.31 - 3.36 (m, 1H), 3.59 (s, 3H), 3.71 (s, 3H), 4.46 - 4.55 (m, 1H), 6.60 - 6.65 (dd, 1H), 6.73 (s, 1H), 6.80 (d, 1H), 7.10 - 7.28 (m, 5H), 8.45 (d, 1H), 8.75 (brs, 1H).
ESI-MS; 487.3 (MH⁺).

### (Example 18)

### Crystallization of N-[N-[3-(3-hydroxy-4-methoxyphenyl)-3-methylbutyl]-L-α-aspartyl]-L-phenylalanine 1-methyl ester, and Separation of the crystals thereof

3-(3-Hydroxy-4-methoxyphenyl)-3-methylbutylaldehyde (0.460 g, 2.21 mmol) and aspartame (0.683 g, 2.32 mmol) were added to a mixed solvent (26 ml) of methanol and water (Mixing ratio of 3:2 v/v), and then the mixture was stirred at room temperature in a short time. 10 % Palladium carbon in the water content of 50 % (0.233 g) was added thereto, and the mixture was stirred under hydrogen atmosphere of normal pressure (0.1 MPa) at room temperature for 48 hours for reaction. Thus obtained reaction solution was heated up to 45 °C and stirred for 30 minutes. After that, thus obtained reaction solution was filtrated to remove the catalyst, and further, the catalyst was washed with methanol (10 ml). The filtrate and the wash solution were combined together to obtain a reaction solution (38 ml) containing N-[N-[3-(3-hydroxy-4-methoxyphenyl)-3-methylbutyl]-L-α-aspartyl]-L-phenylalanine 1-methyl ester (0.795 g, 1.63 mmol). This reaction solution was concentrated under reduced pressure at 40 °C up to approximately one fourth (1/4) of the volume thereof, and then to the concentrated solution while stirring at 50 °C, methanol (1 ml) was added to precipitate crystals. This crystallization solution was cooled to 5 °C and allowed to stand overnight at the same temperature. The crystals were separated by filtration, and further washed with water (20 ml), and dried overnight under reduced pressure at room temperature to obtain crude crystals thereof (0.841 g, Content of the object compound: 0.513 g, Recovery rate: 65 %). Thus obtained crude crystals were added to a mixed solvent (26 ml) of methanol and water (Mixing ratio of 3:2 v/v) and dissolved therein at 60 °C. After that, the solution was cooled to 5 °C to precipitate crystals. The mixture was allowed to stand overnight at the same temperature, and then the crystals were separated by filtration, and washed with water in small quantities, and dried under reduced pressure at 40 °C for 4 hours to obtain N-[N-[3-(3-hydroxy-4-methoxyphenyl)-3-methylbutyl]-L-α-aspartyl]-L-phenylalanine 1-methyl ester (0.402 g, 0.826 mmol, Crystallization yield: 78 %).

The purity in the high performance liquid chromatography (HPLC) analysis was not less than 97 %.

### (Example 19)

### Crystallization of N-[N-[3-(3-hydroxy-4-methoxyphenyl)-3-methylbutyl]-L-α-aspartyl]-L-phenylalanine 1-methyl ester, and Separation of the crystals thereof

3-(3-Hydroxy-4-methoxyphenyl)-3-methylbutylaldehyde (6.01 g, 28.8 mmol) and aspartame (9.89 g, 33.6 mmol) were added to a mixed solvent (330 ml) of methanol and water (Mixing ratio of 3:2 v/v), and then the mixture was stirred at 25 °C in a short time. 10 % Palladium carbon in the water content of 50 % (3.46 g) was added thereto, and the mixture was stirred under hydrogen atmosphere of normal pressure (0.1 MPa) at 25 °C for 47 hours for reaction. Thus obtained reaction solution was heated up to 45 °C and stirred for 30 minutes. After that, thus obtained reaction solution was filtrated to remove the catalyst, and further, the catalyst was washed with a mixed solvent (150 ml) of methanol and water (Mixing ratio of 3: 2 v/v). The filtrate and the wash solution were combined together to obtain a reaction solution (439 g) containing N-[N-[3-(3-hydroxy-4-methoxyphenyl)-3-methylbutyl]-L-α-aspartyl]-L-phenylalanine 1-methyl ester (9.32 g, 19.2 mmol). To this reaction solution, toluene (288 ml) was added, and thus obtained solution was stirred at room temperature for 30 minutes to make layers separated therein. The toluene layer was removed therefrom, and thereby the aqueous layer (458 g) was obtained.

This aqueous layer was concentrated under reduced pressure at 40 °C up to an amount of residual solution of 224 g, and heated up to 75 °C, and then cooled to 5 °C while stirring (Cooling speed: 10 °C/hour) to precipitate crystals. Thus obtained mixture was stirred at the same temperature for 16 hours. After that, the crystals were separated by filtration, and further washed with water (20 ml) to obtain the wet crude crystals (14.0 g, Content of the object compound: 8.61 g, Recovery rate: 92 %). To thus obtained wet crude crystals, a mixed solvent (900 ml) of methanol and water (Mixing ratio of 2 : 3 v/v) was added, and the crystals were dissolved therein at 75 °C. The solution was cooled while stirring to 5 °C (Cooling speed: 10 °C/hour) to precipitate crystals. The mixture was stirred at the same temperature for 65 hours, and thereafter the crystals were separated by filtration, and further washed with a mixed solvent (20 ml) of methanol and water (Mixing ratio: 2 : 3 v/v), and dried under reduced pressure at room temperature for 5 hours to obtain N-[N-[3-(3-hydroxy-4-methoxyphenyl)-3-methylbutyl]-L-α-aspartyl]-L-phenylalanine 1-methyl ester (6.15 g, 12.6 mmol, Crystallization yield: 71 %).

### (The purity in the HPLC analysis was not less than 98 %.)

### (Example 20)

### Crystallization of N-[N-[3-(3-hydroxy-4-methoxyphenyl)-3-methylbutyl]-L-α-aspartyl]-L-phenylalanine1-methyl ester, and Separation of the crystals thereof

3-(3-Hydroxy-4-methoxyphenyl)-3-methylbutylaldehyde (0.423 g, 2.03 mmol) and aspartame (0.683 g, 2.32 mmol) were added to a mixed solvent (26 ml) of methanol and water (Mixing ratio of 3:2 v/v), and then the mixture was stirred at room temperature in a short time. 10 % Palladium carbon in the water content of 50 % (0.233 g) was added thereto, and the mixture was stirred under hydrogen atmosphere of normal pressure (0.1 MPa) at room temperature for 48 hours for reaction. Thus obtained reaction solution was heated up to 45 °C and stirred for 30 minutes. After that, thus obtained reaction solution was filtrated to remove the catalyst, and further, the catalyst was washed with methanol (10 ml). The filtrate and the wash solution were combined together to obtain a reaction solution (32.6 g) containing N-[N-[3-(3-hydroxy-4-methoxyphenyl)-3-methylbutyl]-L-α-aspartyl]-L-phenylalanine 1-methyl ester (0.988 g, 2.03 mmol).

This reaction solution was concentrated under reduced pressure at 40 °C up to an amount of residual solution of 10.1 g, and heated up to 75 °C, and then cooled to 5 °C while stirring (Cooling speed: 10 °C/hour) to precipitate crystals. Thus obtained mixture was stirred overnight at the same temperature. After that, the crystals were separated by filtration, and further washed with water (2.6 ml) to obtain the wet crude crystals (1.31 g, Content of the object compound: 0.909 g, Recovery rate: 92 %). To thus obtained wet crude crystals, a mixed solvent (52 ml) of methanol and water (Mixing ratio of 1 : 1 v/v), and toluene (26 ml) were added. Thus obtained mixture was stirred at room temperature for 10 minutes to make layers separated therein. The toluene layer was removed, and thereby the aqueous layer was obtained, and then was concentrated under reduced pressure at 40 °C to obtain a concentrated solution (24.1 g). To this concentrated solution, methanol (4 ml) was added, and the insoluble material was dissolved therein at 75 °C. Thus obtained solution was cooled to 5 °C while stirring (Cooling speed: 10 °C/hour) to precipitate crystals. After the mixture was stirred for 40 hours at the same temperature, the crystals were separated by filtration, and further washed with a mixed solvent (3 ml) of methanol and water (Mixing ratio: 1 : 4 v/v), and dried under reduced pressure at room temperature for 5 hours to obtain N-[N-[3-(3-hydroxy-4-methoxyphenyl)-3-methylbutyl]-L-α-aspartyl]-L-phenylalanine 1-methyl ester (0.617 g, 1.268 mmol, Crystallization yield: 68 %).

### (The purity in the HPLC analysis was not less than 98 %.)

### (Example 21)

### Physical properties on N-[N-[3-(3-hydroxy-4-methoxyphenyl)-3-methylbutyl] -L-α-aspartyl]-L-phenylalanine 1-methyl ester in the crystalline form

The physical properties on the crystals of the title compound obtained in the present invention were in the followings.
Differential thermal analysis:
Temperature range for the determination: 50-300 °C; Heating-up speed: 10 °C/minute; Melting point: 189 °C.
Powder X-ray diffraction:

As shown in the figure 1, the characteristic peaks were observed (exhibited) in the diffraction angles of 8.3°, 19.5° and 21.2° (2θ, CuKα ray).

### Effect of Invention

According to the present invention, aspartame is alkylated reductively with 3-(phenyl with substituent group(s))-3-methylbutylaldehyde to be able to produce N-[N-[3-(phenyl with substituent group(s))-3-methylbutyl]-L-α-aspartyl]-L-phenylalanine 1-methyl ester derivative efficiently on an industrial scale, which is excellent as a sweetener having a high degree of sweetness and therefore is an objective compound described above.

Further, by using a novel 3-(3-hydroxy-4-methoxyphenyl)-3-methylbutylaldehyde used for an intermediate in the production of sweetener in the present invention, N-[N-[3-(3-hydroxy-4-methoxyphenyl)-3-methylbutyl]-L-α-aspartyl]-L-phenylalanine 1-methyl ester which is important as a sweetener having a high degree of sweetness, can be easily produced efficiently on an industrial scale.

The above 3-(3-hydroxy-4-methoxyphenyl)-3-methylbutyl aldehyde can be produced easily and efficiently in a process for subjecting 3-(3-halogeno-4-methoxyphenyl)-3-methylbutyric acid, which can be obtained in the reaction of 2-halogenoanisole with 3-methylcrotonic acid, to a reaction for converting the halogen atom of a substituent group to a hydroxyl group to produce 3-(3-hydroxy-4-methoxyphenyl)-3-methylbutyric acid, followed by converting the carboxyl group of the carboxylic acid thus obtained into a formyl group.

Moreover, in the present invention, N-[N-[3-(3-hydroxy-4-methoxyphenyl)-3-methylbutyl]-L-α-aspartyl]-L-phenylalanine 1-methyl ester which is important as a sweetener having a high degree of sweetness, can be crystallized, and thus the compound can be obtained easily and conventionally in the crystalline form. Further, the present invention can be also used for a process to separate the object compound only with high purity through purification from the object compound containing impurity, and therefore, the present invention is useful industrially to an extreme.

Since a high purity of material (crystals) can be obtained and provided, the compound as such can be used directly as a sweetener component, and the compound can be easily used for a food and drink, or the like in need of a sweet taste.

## Claims

1. A process for producing aspartyl dipeptide ester derivative represented by the following general formula (2), which comprises:
alkylating reductively aspartame with the aldehyde represented by the following general formula (1): 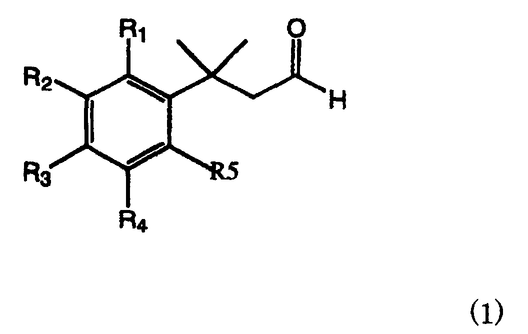 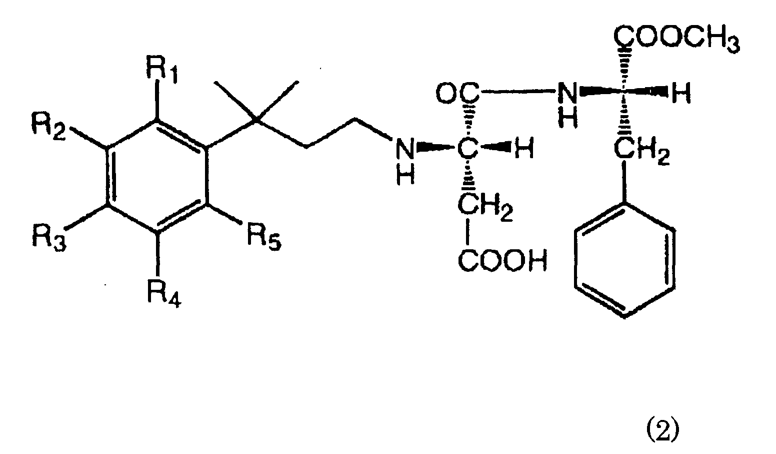
In the above formulae (1) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, a benzyloxy group and a hydroxyalkyloxy group having 2 or 3 carbon atoms, wherein two symbols of R₁ and R₂, or two symbols of R₂ and R₃ may be combined together to denote a methylene dioxy group, and
provided that in the formula (2), any one of R₁, R₂, R₃, R₄ and R₅ does not denote a benzyloxy group.

2. The process as defined in claim 1, wherein in the formulae, R₃ is a methoxy group, and R₁, R₂, R₄ and R₅ are a hydrogen atom.

3. The process as defined in claim 1, wherein in the formulae, R₃ is a hydroxyl group, and R₁, R₂, R₄ and R₅ are a hydrogen atom, and in the formula (1), R₃ may be a benzyloxy group.

4. The process as defined in claim 1, wherein in the formulae, R₂ is a methoxy group, R₃ is a hydroxyl group, and R₁, R₄ and R₅ are a hydrogen atom, and in the formula (1), R₃ may be a benzyloxy group.

5. The process as defined in claim 1, wherein in the formulae, R₂ is a hydroxyl group, R₃ is a methoxy group, and R₁, R₄ and R₅ are a hydrogen atom, and in the formula (1), R₂ may be a benzyloxy group.

6. The process as defined in claim 1, wherein in the formulae, R₁ is a hydroxyl group, and R₂, R₃, R₄ and R₅ are a hydrogen atom, and in the formula (1), R₁ may be a benzyloxy group.

7. The process as defined in claim 1, wherein in the formulae, R₁ is a hydroxyl group, R₃ is a methoxy group, and R₂, R₄ and R₅ are a hydrogen atom, and in the formula (1), R₁ may be a benzyloxy group.

8. The process as defined in claim 1, wherein in the formulae, R₁ is a hydroxyl group, R₃ is a methyl group, and R₂, R₄ and R₅ are a hydrogen atom, and in the formula (1), R₁ may be a benzyloxy group.

9. The process as defined in claim 1, wherein in the formulae, R₂ and R₃ are combined together to denote a methylene dioxy group, and R₁, R₄ and R₅ are a hydrogen atom.

10. The process as defined in claim 1, wherein in the formulae, R₂ is a methyl group, R₃ is a methoxy group, and R₁, R₄ and R₅ are a hydrogen atom.

11. The process as defined in claim 1, wherein in the formulae, R₂ is a methyl group, R₃ is a hydroxyl group, and R₁, R₄ and R₅ are a hydrogen atom, and in the formula (1), R₃ may be a benzyloxy group.

12. The process as defined in claim 1, wherein in the formulae, R₂ is a hydroxyl group, R₃ is a methyl group, and R₁, R₄ and R₅ are a hydrogen atom, and in-the formula (1), R₂ may be a benzyloxy group.

13. The process as defined in claim 1, wherein the reaction for alkylating reductively said aspartame is conducted in the presence of catalyst for hydrogenation.

14. The process as defined in claim 13, wherein said catalyst is palladium carbon or platinum carbon.

15. The process as defined in claim 1, wherein the reaction for alkylating reductively said aspartame is conducted in a solvent, and said solvent is an alcohol or a water-containing alcohol.

16. A process for producing 3-(3-hydroxy-4-methoxyphenyl)-3-methylbutylaldehyde, which comprises:
subjecting 3-(3-hydroxy-4-methoxyphenyl)-3-methylbutyric acid to a reaction for converting a carboxyl group into a formyl group.

17. The process as defined in claim 16, wherein said 3-(3-hydroxy-4-methoxyphenyl)-3-methylbutyric acid is obtained by any one step of the following steps:
a. subjecting 3-(3-halogeno-4-methoxyphenyl)-3-methylbutyric acid to a reaction for converting a halogen atom of the substituent group into a hydroxyl group; and
b. subjecting 3-(3-halogeno-4-methoxyphenyl)-3-methylbutyric acid obtained through the step of reaction of 2-halogenoanisole with 3-methylcrotonic acid,
to a reaction for converting a halogen atom of the substituent group into a hydroxyl group.

18. The process as defined in claim 17, wherein in said 3-(3-halogeno-4-methoxyphenyl)-3-methylbutyric acid, the halogen atom located at the 3-positon of the phenyl group is a chlorine atom or a bromine atom.

19. The process as defined in claim 17, wherein said reaction with 3-methylcrotonic acid is conducted in the presence of an acid.

20. The process as defined in claim 16, wherein said reaction for converting a carboxyl group into a formyl group is any one of the following reactions:
a. a reaction for converting a carboxylic acid to an aldehyde through a half reduction thereof; and
b. a reaction for converting a carboxyl group into a hydroxymethyl group, and thereafter converting it into a formyl group.

21. A process for producing N-[N-[3-(3-hydroxy-4-methoxyphenyl)-3-methylbutyl]-L-α-aspartyl]-L-phenylalanine 1-methyl ester, which comprises:
subjecting 3-(3-hydroxy-4-methoxyphenyl)-3-methylbutyl aldehyde obtained in the process as defined in any one of claims 16 to 20 and aspartame to a reductive alkylation reaction.

22. A process for producing N-[N-[3-(3-hydroxy-4-methoxyphenyl)-3-methylbutyl]-L-α-aspartyl]-L-phenylalanine 1-methyl ester, which comprises:
subjecting 3-(3-hydroxy-4-methoxyphenyl)-3-methylbutyl aldehyde and aspartame to a reductive alkylation reaction.

23. A benzene derivative represented by the following general formula (3): 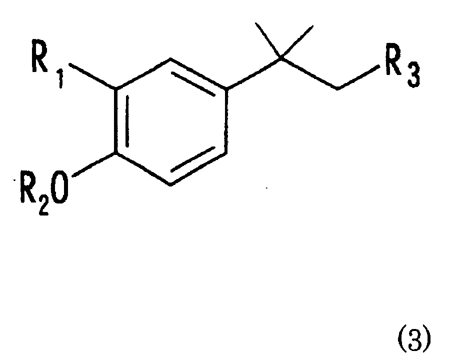
In the above formula, R₁ denotes any one substituent group of a hydroxyl group, a halogen atom and a lower alkyloxy group having 1 to 4 carbon atoms, R₂ denotes a lower alkyl group having 1 to 4 carbon atoms, and R₃ denotes any one substituent group of a carboxyl group, a formyl group and a hydroxymethyl group,
provided that the compounds where R₁ is a chlorine atom or a bromine atom, and R₃ is a formyl group are excluded.

24. A benzene derivative selected from the following compounds a. to e.:
a. 3-(3-hydroxy-4-methoxyphenyl)-3-methylbutylaldehyde;
b. 3-(3-chloro-4-methoxyphenyl)-3-methylbutyric acid;
c. 3-(3-bromo-4-methoxyphenyl)-3-methylbutyric acid;
d. 3-(3-hydroxy-4-methoxyphenyl)-3-methylbutyric acid; and
e. 3-(3-hydroxy-4-methoxyphenyl)-3-methyl-1-butanol.

25. A process for producing N-[N-[3-(3-hydroxy-4-methoxyphenyl)-3-methylbutyl]-L-α-aspartyl]-L-phenylalanine 1-methyl ester, which comprises:
subjecting N-[N-[3-(3-hydroxy-4-methoxyphenyl)-3-methylbutyl]-L-α-aspartyl]-L-phenylalanine 1-methyl ester containing impurity to a crystallization step to crystallize said compound.

26. The process as defined in claim 25, wherein said N-[N-[3-(3-hydroxy-4-methoxyphenyl)-3-methylbutyl]-L-α-aspartyl]-L-phenylalanine 1-methyl ester containing impurity is a material obtained by subjecting aspartame and 3-(3-hydroxy-4-methoxyphenyl)-3-methylbutylaldehyde or its derivative to a reductive alkylation reaction.

27. The process as defined in any one of claims 25 and 26, wherein said impurity includes at least one compound selected from the group consisting of aspartame, aspartame derivative, peptide derivative, amino acid, amino acid derivative, aldehyde and its derivative.

28. The process as defined in any one of claims 25 to 27, wherein the solvent used for said crystallization step is at least one solvent selected from the group consisting of methanol, ethanol, isopropyl alcohol, acetone, methyl ethyl ketone, methyl isobutyl ketone, methyl acetate, ethyl acetate, propyl acetate, isopropyl acetate, butyl acetate, tetrahydrofuran, acetonitrile and toluene, or a mixed solvent of at least one solvent of these organic solvents, with water.

29. The process as defined in any one of claims 25 to 28, which further comprises a step for removing said impurity through an extraction process with a solvent.

30. The process as defined in claim 29, wherein the solvent used for said extraction process with a solvent is at least one solvent selected from the group consisting of toluene, diethyl ether, chloroform, dichloromethane, hexane, ethyl acetate, propyl acetate, isopropyl acetate and butyl acetate.

31. A crystal of N-[N-[3-(3-hydroxy-4-methoxyphenyl)-3-methylbutyl]-L-α-aspartyl]-L-phenylalanine 1-methyl ester, which exhibits peaks of diffractive X-ray in at least diffraction angles of 8.3°, 19.5° and 21.2° (2θ, CuKα ray) when determined in the powder X-ray diffractometry.

32. The crystal as defined in claim 31, which is obtained in the process containing the process as defined in any one of claims 25 to 30.

33. A sweetener, or a food and drink or other product with a sweet taste imparted, comprising the crystal as defined in claim 31 as an effective ingredient, which may further comprise a carrier or a bulking agent for sweeteners.

34. A process for imparting a sweet taste, which comprises:
adding the crystal as defined in claim 31 to or including said crystal in a product such as a food and drink or the like in need of a sweet taste imparted or the intermediate product therefor during manufacture.
